# NEUE EUROPÄISCHE PATENTSCHRIFT

(11) **EP 0 379 133 B2**
(45) Veröffentlichungstag und Bekanntmachung des Hinweises auf die Entscheidung über den Einspruch: **16.07.2003**
(45) Hinweis auf die Patenterteilung: 02.08.1995
(21) Anmeldenummer: 90100800.3
(22) Anmeldetag: 16.01.1990
(51) Int. Cl.: G01N 33/53, G01N 33/543

(54) **Verwendung von zweiwertigen Kationen in immunchemischen Tests**
Use of divalent cations in immunochemical tests
Utilisation de cations divalents dans les tests immunochimiques

(30) Priorität: 19.01.1989 DE 3901458
(43) Veröffentlichungstag der Anmeldung: 25.07.1990
(73) Patentinhaber: Dade Behring Marburg GmbH, 35041 Marburg (DE)
(72) Erfinder: Giesendorf, Bernhard, Dr., D-6255 Dornburg-Frickhofen (DE)

(56) Entgegenhaltungen:
- WO-A-90/02556
- DE-A- 3 609 217
- US-A- 4 362 531
- Journal of Clinical Microbiology, 1986 s.405-413

## Beschreibung

Die Erfindung betrifft die Verwendung von zweiwertigen Kationen in einem Festphasen-ELISA zum Nachweis und zur Bestimmung eines Analyten in biologischem Material.

Bekannte immunchemische Testsysteme gebrauchen Zusätze von Proteinen, Polysacchariden und/oder Tensiden, die nicht an der immunchemischen Reaktion beteiligt sind, die jedoch geeignet sind, das Ergebnis einer solchen Reaktion günstig zu beeinflussen.

Für Festphasen-ELISAs, die eine Auswahl solcher Testsysteme darstellen, müssen die erforderlichen Inkubationsmedien (Pufferlösungen, Inkubationsmilieus), die den Analyten enthalten und die mit der Festphase zusammengebracht werden, so zusammengesetzt sein, daß die unspezifische Bindung von Begleitsubstanzen der Probe und/oder des Konjugats an die Festphase verhindert wird. Deshalb werden in den Inkubationsmedien die bekannten Zusätze, wie Proteine, z.B. Albumin, IgG, Kasein, hydrolisierte Gelatine und deren Derivate und Mischungen von Proteinen oder auch humane oder tierische Seren sowie Tenside verwendet.

In EP 0 152 847 werden Enzym-Antigen und Enzym-Antikörper-Konjugate beschrieben, die Calciumsalze und Polyoxiethylen zusammen enthalten, wobei die Haltbarkeit eines Konjugats in wässriger Lösung durch den Zusatz beider Stoffe jedoch nicht durch jeden der Stoffe alleine erhöht wird.

In DE 36 38 767 ist ein Inkubationsmedium für festphasenimmunchemische Tests, beispielsweise einen ELISA, beschrieben, das Lactoferrin, foetales Kälberserum, Polyoxiethylen-20-sorbitanmonolaureat (^{R}Tween 20) und Puffersalze enthält.

In DE 38 07 478 sind Aminoxide als vorteilhafter Zusatz bei immunchemischen Mitteln, insbesondere bei in ihnen enthaltenen Inkubationsmedien genannt.

In US 4 362 531 wird die Verbesserung der Spezifität von Partikel-Agglutinations-Immunoassays durch chaotrope oder chaotrop-öhnliche Agentien beschrieben.

In abstract 86-159 165 zur japanischen Patent-anmeldung JP-61-159 165 wird die Stabilisierung von Polymersuspensionen mit Calciumpantothenat gezeigt, ohne Angabe einer geeigneten Konzentration.

In MIDDELDORP et al, J. Clin. Microbiology, 1986, 405-413 werden spezifische Immunofluoreszenztests zum Nachweis virusspezifischer Antikörper in Gegenwart von 2,6 mM Ca^{2⊕} und 2,2 mM Mg^{2⊕} beschrieben.

Es wurde nun überraschend gefunden, daß zweiwertige Kationen als Zusatz zu Mitteln, mit denen ein Analyt in einer Probe durch eine immunchemische Reaktion nachgewiesen oder bestimmt wird, insbesondere in Kombination mit den zuvor genannten Zusätzen geeignet sind, die immunchemische Reaktion günstig zu beeinflussen, was eine höhere Spezifität des Nachweises und der Bestimmung des Analyten bewirkt

Gegenstand der Erfindung ist daher die Verwendung von zweiwertigen Kationen, vorzugsweise von wasserlöslichen Magnesium- und/oder Calziumsalzen in einem Festphasen-ELISA züm Nachweis und zur immunchemischen Bestimmung eines Analyten enthalten in einem biologischen Material, dadurch gekennzeichnet, daß die entsprechenden Salze beim Zusammenbringen des Analyten mit einem nicht markierten Reaktanten in einer Konzentration von 5 bis 500 mmol/l in gelöster Form vorliegen.

Aus der Gruppe der wasserlöslichen Magnesium- oder Calciumsalze werden solche bevorzugt, deren Anionen die immunchemische Reaktion nicht stören. Bevorzugt werden als Anionen Acetat, Chlorid und Citrat, besonders bevorzugt Chlorid.

Verfahren im Sinne der Erfindung sind solche, bei denen die immunchemische Reaktion an einer Festphase stattfindet.

Ein Festphase ist dabei ein wasserunlöslicher Träger, an dem ein oder mehrere Reaktanten gebunden ist. Der Analyt kann entweder an einen Reaktanten binden oder im Falle des Vorhandenseins von mehreren an den Träger gebundenen Reaktanten mindestens einen davon durch die eigene Bindung lösen und in die wässrige Phase abgeben.

In immunchemischen Verfahren werden Antigen und Antikörper sowohl als Analyten als auch als Reaktanten sowie andere für die Reaktanten oderauch den Analyten bioaffine Bindungspartner, beispielsweise Lektine, Komplement, Protein A oder G sowie derivatisiertes Biotin und Avidin verwandt.

Die zweiwertigen Kationen können in einer Vorrichtung zurAufnahme einer Probe, beispielsweise in einem Gefäß zur Probenaufnahme oder in einer Aufnahmezone, beispielsweise einer saugfähigen Matrix, für die Probe auf einem sogenannten "trockenchemischen" Testsystem in trockener Form enthalten sein; oder auch in einer wässrigen Lösung, in der auch Puffersalze und ein Detergenz und gegebenenfalls stabilisierende Zusätze wie Proteine oder Polysaccharide als ebenfalls für den Analyten stabilisierende Substanzen enthalten sind, wobei die zwei-wertigen Kationen in einer Konzentration von 5 bis 500 mmol/l enthalten sind, bevorzugt von 30 bis 100 mmol/l, besonders bevorzugt von 50 bis 60 mmol/l.

Die auch als Probe bezeichneten biologischen Materialien, die den Analyten enthalten, sind beispielsweise Gewebe von Biopsien oder Autopsien, Blutzellen, Serum oder Plasma, Sekrete, Liquor, Blut aus entzündetem und nicht entzündetem Gewebe, die Zerfallsprodukte von Gewebe und Stoffwechselausscheidungen.

Bevorzugt werden immunchemische Verfahren, bei denen einer der Reaktanten in fester Phase vorliegt, wobei die Probe in zweiwertigen Kationen mit der festen Phase zusammengebracht wird, gegebenenfalls zusammen mit weiteren immunchemischen Reaktanten außer denen der festen Phase und Reagenzien zum Nachweis des Analyten, wobei anschließend die feste Phase von der flüssigen Phase getrennt und entweder der an der festen Phase gebundene Analyt oder der ungebundene Analyt bestimmt wird.

In den folgenden Beispielen wird die Vorteilhaftigkeit der Verwendung von zweiwertigen Kationen in Verfahren zum Nachweis und zur Bestimmung von Antikörpern, gerichtet gegen den Erreger der (1) infektiösen bovinen Rhinotracheitis (IBR), bzw. der infektiösen pustulären Vulvovaginitis (IPV) und (2) Rinderleukose dargelegt.

### Beispiele

### Beispiel 1

ELISA zum Nachweis von Antikörpern gegen den Erreger von IBR und von IPV

### 1.1 Puffer- und Reagenzlösungen

Folgende Puffer- und Reagenzlösungen wurden hergestellt
1.1.1 PBS-^{R}Tween 20
   Phosphatgepufferte physilogische Kochsalzlösung, pH 7.2 (PBS), d.h. 10 mmol/l Na₂HPO₄/KH₂PO₄, pH 7.2 in 140 mmol/l NaCl, enthaltend 10 g/l Polyoxiethylen-20-sorbitan-monolaureat (^{R}Tween 20).
1.1.2 Probenverdünnungspuffer (PT)
   PBS enthaltend 40 g/l ^{R}Tween 20.
1.1.3 Probenverdünnungspuffer enthaltend 50 mmol/l MgCl₂ (PT-Mg)
   10 g/l MgCl₂ x 6H₂O wurden in PT gelöst.
1.1.4 Tris/EDTA
   50 mmol/l Tris, 50 mmol/l
   Ethylendiamintetraessigsäure-dinatriumsalz als wässrige Lösung, eingestellt mit HCI auf pH 7.2.
1.1.5 AP-Konjugat
   Das unter der Produkt Nr. OUDY01 erhältliche, als Anti-Rind-Immunglobulin-Alkalische Phosphatase-Konjugat bezeichnete Reagenz der Behringwerke AG wurde 1:70 mit PT verdünnt
1.1.6 AP-Substratlösung
   1.5 g/l p-Nitrophenylphosphat in 100 g/l Diethanolamin in Wasser, eingestellt mit HCI auf pH 9.5.

### 1.2 Beschichtung von Mikrotestplatten mit bovinem Herpesvirus I (BHVI)

Es wurden als Mikrotestplatten Immunoplatten II 96 F mit rundem Boden (Firma Nunc, Roskilde, Dänemark, Artikel Nr. 262162) verwandt.

Gewebekulturen von Madin-Darby-Bovine-Kidney(Niere)-Zellen (MDBK-Zellen) in denen BHVI vermehrt worden waren und Gewebekulturen die kein BHVI enthielten, wurden, wie von Nicolai-Scholten et al. 1980 in Med.Microbiol.Immunol. 168, 81-90 für Mumps-Virus beschrieben, zu Präparationen aufgearbeitet, die im weiteren als BHVI-Antigen (Ag) und als (negatives) Kontroll-Antigen (KoAg) bezeichnet werden.

Zur Beschichtung wurden jeweils 50 µl BHVI-Antigen (Ag) und KoAg in die Vertiefungen der obengenannten Mikrotestplatten alternierend gegeben und die Platten 20 h bei 20-25°C stehengelassen. Anschließend wurde der Inhalt der Vertiefungen durch Absaugen entfernt und die Vertiefungen einmal mit PBS-Tween^{R}20 und zweimal mit Tris/EDTA durch Füllen und anschließendem Absaugen gewaschen.

### 1.3 Verfahren zur Bestimmung von IgM, IgG-Antikörpern gerichtet gegen den Erreger von IBR/IPV

Positive Kontrollseren wurden mit den Puffern PT und PT-Mg in einer Verdünnungsreihe von 1:40 bis 1:640 verdünnt.

Serumproben wurden 1:44 mit den obengenannten Puffern verdünnt.

Von jeder Verdünnung wurden 150 µl in Vertiefungen einer Mikrotestplatte, beschichtet mit Ag und KoAg, gegeben. Die Testplatte wurde dann 1 h bei 37°C bei gesättigter Luftfeuchtigkeit gehalten. Anschließend wurden die Vertiefungen dreimal mit PBS-^{(R)}Tween durch Füllen der Vertiefungen und Absaugen der Flüssigkeit gewaschen. Dann wurde 50 µl AP-Konjugat zugegeben und nochmals 1 h bei 37°C stehen gelassen und anschließend gewaschen wie oben beschrieben. Zur Bestimmung der Aktivität der in den Vertiefungen als Konjugat gebundenen AP wurden 100 µl AP-Substratlösung zugegeben, die Mikrotestplatte 45 min bei 37°C stehen gelassen und die Extinktion der gelb gefärbten Lösungen gegen eine leere Vertiefung bei 405 nm gemessen.

Die Seren wurden als positiv bewertet, wenn die Differenz der Extinktionen von den gefärbten Lösungen in mit Ag beschichteten und von Lösungen in mit KoAg beschichteten Vertiefungen größer als plus 200 milli-Extinktionen (mE) und als negativ bewertet, wenn die Differenz kleiner als plus 200 mE oder negativ war.

Als Titer eines Kontrollserums wurde die Verdünnung angenommen, bei der diese Differenz kleiner als 200 mE wurde. Der Wert von 200 mE wird deshalb als "cut off" bezeichnet.

### 1.4 Ergebnis

Das Ergebnis der Messung ist in der Tabelle dargestellt Aus ihr wird ersichtlich, daß der Zusatz von MgCl₂ zu dem Probenverdünnungspuffer PT bei den beiden 1:640 verdünnten Kontrollseren keine Unterschiede in den Extinktionen, weder bei den mit Ag, noch bei mit KoAg beschichteten Vertiefungen bewirkt Jedoch ist bei den 1:44 verdünnten Seren der Zusatz von MgCl₂ vorteilhaft insofern, als die Absolutwerte der Extinktionen sowohl bei den mit Ag als auch mit KoAg beschichteten Vertiefungen niedriger sind als die Absolutwerte der Extinktionen bei Verwendung des Puffers ohne die genannten Zusätze. Die Erniedrigung der Extinktionen, insbesondere bei den Vertiefungen, die mit KoAg beschichet sind, bedeutet eine Verminderung der Hintergrundextinktionen und somit eine Steigerung der Spezifität des Tests.

### Beispiel 2

ELISA zum Nachweis von Antikörpern gegen den Erreger der Rinderleukose

### 2.1 Pufferlösungen

Es wurden folgende Probenverdünnungspuffer hergestellt
2.1.1 STD
   10 ml/l foetales Kälberserum, 18 mmol/l Trinatriumcitrat x 2H₂O, 10 mmol/l KCI, 40 g/l 1-Alkoyl (C₈ bis C₁₈) amino-3-dimethyl-aminopropan-3-N-oxid in wässriger Lösung.
2.1.2 STD-Mg
   In dem unter 2.1 beschriebenen Probenverdünnungspuffer wurden 50 mmol/l MgCl₂, d. h. 10 g/l MgCl₂ x 6H₂O gelöst.
2.1.3 STD-Ca
   In dem unter 2.1 beschriebenen Probenverdünnungspuffer wurden 68 mmol/l CaCl₂, d. h. 10 g/l CaCl₂ x 2H₂O gelöst.

### 2.2 Beschichtung von Mikrotestplatten mit bovinem Leukämievirus (BLV)

Es wurden dieselben Mikrotestplatten verwandt wie in Beispiel 1.2 beschrieben. BLV wurde auf einer permanenten foetalen Schafsnierenzelle vermehrt und zu Präparationen von BLV-Antigen aufgearbeitet wie von Nicolai-Scholten et al. 1980 in Med.Microbiol.Immunol. 168, 81-90 für Mumps-Virus beschrieben.
Zur Beschichtung wurden jeweils 50 µl BLV-Antigen in die Vertiefungen gegeben. Auf eine Beschichtung mit einer Präparation aus virusfreien Schafsnierenzellen wurde verzichtet. Anschließend wurde der Inhalt der Vertiefungen durch Absaugen entfernt und die Vertiefungen einmal mit PBS-^{(R)}Tween und zweimal mit Tris/EDTA durch Füllen und anschließendes Absaugen gewaschen.

### 2.3 Verfahren zur Bestimmung von IgM- und IgG-Antikörpern, gerichtet gegen BLV

Von BLV-positiven Rindersera wurden Verdünnungen von 1:20 mit den Puffern STD, STD-MgCl₂ und STD-CaCl₂ hergestellt.
Von diesen Verdünnungen wurden 150 µl in die mit BLV-Antigen beschichteten Vertiefungen der in Beispiel 2.2 beschriebenen Mikrotestplatte pipettiert Die Mikrotestplatte wurde weiterbehandeitwe in Beispiel 1.3 beschrieben.

### 2.4 Ergebnis

Es zeigte sich, daß die beiden Analyt-negativen Sera in STD eine Extinktion von 0,46 E bzw. 0,48 E erbrachten. In STD-Mg bzw. STD-Ca erniedrigte sich dieser Wert auf 0,14 E bzw. 0,22 E, d.h. um 69,6% bzw. 54,2%. Demgegenüber erreichte ein Rinderserum, das BLV-spezifische Antikörper enthält in STD eine Extinktion von 1,28 E, in STD-Mg von 1,06 E, in STD-Ca von 1,02 E; der in STD erreichte Wert reduzierte sich hier also lediglich um 17,2% bzw. 20,3%.

Betrachtet man die Ergebnisse zusammenhängend, so zeigt sich, daß unter Einfluß von MgCl₂ bzw. CaCl₂ im Probenverdünnungsmedium die Differenz zwischen den Extinktionen Analyt-positiver und -negativer Sera größer wird, d.h. die Spezifität des Testsystems wird entscheidend verbessert.

**Tabelle**

| | Probenverdünnungspuffer | | | |
|---|---|---|---|---|
| | PT | | PT-Mg | |
| | OD_{Ag} (mE) | OD_{KoAg} (mE) | OD_{Ag} (mE) | OD_{KoAg} (mE) |
| IBR/IPV-Ktr.-Serum, pos., (Verdünnung 1:640) | | | | |
| Charge 16 32 08 | 181 | 43 | 172 | 45 |
| Charge 16 32 09 | 124 | 36 | 119 | 43 |
| | | | | |
| Negative Rinderseren (Verdünnung 1:44) | 559 | 517 | 333 | 365 |
| | 472 | 485 | 324 | 262 |
| | 432 | 429 | 309 | 348 |
| | 703 | 722 | 529 | 537 |
| | 681 | 713 | 395 | 452 |
| | 198 | 161 | 142 | 97 |
| | 333 | 256 | 184 | 152 |
| | 861 | 867 | 675 | 674 |
| | 581 | 636 | 474 | 470 |
| | 493 | 503 | 374 | 387 |
| | 450 | 496 | 357 | 397 |
| | 768 | 819 | 501 | 583 |
| | 311 | 274 | 226 | 176 |
| | 893 | 978 | 758 | 781 |

## Patentansprüche (Patentansprüche für folgende(n) Vertragsstaat(en): AT, BE, CH, DE, FR, GB, IT, LI, LU, NL, SE)

1. Verwendung von zweiwertigen Kationen in einem Verfahren zum immunchemischen Nachweis und zur immunchemischen Bestimmung eines Analyten enthalten in einem biologischen Material zur Verbesserung der Spezifität der immunchemischen Reaktion, **dadurch gekennzeichnet, daß** zweiwartige Kationen, vorzugsweise ein Calcium- und/oder Magnesiumsalz, beim Zusammenbringen des Analyten mit einem nichtmarkierten Reaktanten in einer Konzentration von 5-500 mM/l in gelöster Form vorliegen, wobei besagtes Verfahren ein Festphasen-ELISA ist.

2. Verwendung nach Anspruch 1, **dadurch gekennzeichnet, daß** das Salz das Chlorid ist.

3. Verwendung nach Anspruch 1, **dadurch gekennzeichnet, daß** der Analyt ein Antigen ist.

4. Verwendung nach Anspruch 1, **dadurch gekennzeichnet, daß** der Analyt ein Antikörper ist.

5. Verwendung nach Anspruch 1, **dadurch gekennzeichnet, daß** ein Reaktant als Festphase vorliegt

6. Verwendung nach Anspruch 1, **dadurch gekennzeichnet, daß** das Salz in trockener Form in einer saugfähigen Matrix enthalten ist und in dem immunchemischen Verfahren durch die flüssige Probe oder einer anderen Flüssigkeit in Lösung gebracht wird.

7. Verwendung nach Anspruch 1, **dadurch gekennzeichnet, daß** das Salz zu einer Lösung zugesetzt wird, in der sich der Analyt befindet und mit der der Analyt gegebenenfalls verdünnt wird.

8. Verwendung gemäß mindestens einem der Ansprüche 1 bis 7, **dadurch gekennzeichnet, daß** die zweiwertigen Kationen in einer Konzentration von 30 bis 100 mmol/l vorliegen.

9. Verwendung gemäß mindestens einem der Ansprüche 1 bis 7, **dadurch gekennzeichnet, daß** die zweiwertigen Kationen in einer Konzentration von 50 bis 60 mmol vorliegen.

## Patentansprüche (Patentansprüche für folgende(n) Vertragsstaat(en): ES)

1. Verfahren zum immunchemischen Nachweis und zur immunchemischen Bestimmung eines Analyten enthalten in einem biologischen Material zur Verbesserung der Spezifität der immunchemischen Reaktion, **dadurch gekennzeichnet, daß** zweiwertige Kationen, vorzugsweise ein Calcium- und/oder Magnesiumsalz beim Zusammenbringen des Analyten mit einem nicht markierten Reaktanten in einer Konzentration von 5 bis 500 mmol/l in gelöster Form vorliegen wobei besagtes Verfahren ein Festphasen-ELISA ist.

2. Verfahren nach Anspruch 1, **dadurch gekennzeichnet, daß** das Salz das Chlorid ist.

3. Verfahren nach Anspruch 1, **dadurch gekennzeichnet, daß** der Analyt ein Antigen ist

4. Verfahren nach Anspruch 1, **dadurch gekennzeichnet, daß** der Analyt ein Antikörper ist.

5. Verfahren nach Anspruch 1, **dadurch gekennzeichnet, daß** ein Reaktant als Festphase vorliegt.

6. Verfahren nach Anspruch 1, **dadurch gekennzeichnet, daß** das Salz in trockener Form in einer saugfähigen Matrix enthalten ist und in dem immunchemischen Verfahren durch die flüssige Probe oder einer anderen Flüssigkeit in Lösung gebracht wird.

7. Verfahren nach Anspruch 1, **dadurch gekennzeichnet, daß** das Salz zu einer Lösung zugesetzt wird, in der sich der Analyt befindet und mit der der Analyt gegebenenfalls verdünnt wird.

8. Verfahren gemäß mindestens einem der Ansprüche 1 bis 7, **dadurch gekennzeichnet, daß** die zweiwertigen Kationen in einer Konzentration von 30 bis 100 mmol/l vorliegen.

9. Verfahren gemäß mindestens einem der Ansprüche 1 bis 7, **dadurch gekennzeichnet, daß** die zweiwertigen Kationen in einer Konzentration von 50 bis 60 mmol vorliegen.

## Claims (Claims for the following Contracting State(s): AT, BE, CH, DE, FR, GB, IT, LI, LU, NL, SE)

1. The use of doubly charged cations in a method for the immunochemical detection and for the immunochemical determination of an analyte contained in a biological material for improving the specificity of the immunochemical reaction, wherein doubly charged cations, preferably a calcium and/or magnesium salt, are present in dissolved form in a concentration of 5 to 500 mmol/l when the analyte is brought into contact with an unlabeled reactant, said method being a solid-phase ELISA.

2. The use as claimed in claim 1, wherein the salt is the chloride.

3. The use as claimed in claim 1, wherein the analyte is an antigen.

4. The use as claimed in claim 1, wherein the analyte is an antibody.

5. The use as claimed in claim 1, wherein one reactant is present as solid phase.

6. The use as claimed in claim 1, wherein the salt is contained in dry form in an absorbent matrix and is dissolved in the immunochemical method by the liquid sample or another liquid.

7. The use as claimed in claim 1, wherein the salt is added to a solution which contains the analyte and with which the analyte is diluted where appropriate.

8. The use as claimed in at least one of claims 1 to 7, wherein the doubly charged cations are present in a concentration of 30 to 100 mmol/l.

9. The use as claimed in at least one of claims 1 to 7, wherein the doubly charged cations are present in a concentration of 50 to 60 mmol.

## Claims (Claims for the following Contracting State(s): ES)

1. A method for the immunochemical detection and for the immunochemical determination of an analyte contained in a biological material for improving the specificity of the immunochemical reaction, wherein doubly charged cations, preferably a calcium and/or magnesium salt, are present in dissolved form in a concentration of 5 to 500 mmol/l when the analyte is brought into contact with an unlabeled reactant, said method being a solid-phase ELISA.

2. The method as claimed in claim 1, wherein the salt is the chloride.

3. The method as claimed in claim 1, wherein the analyte is an antigen.

4. The method as claimed in claim 1, wherein the analyte is an antibody.

5. The method as claimed in claim 1, wherein one reactant is present as solid phase.

6. The method as claimed in claim 1, wherein the salt is contained in dry form in an absorbent matrix and is dissolved in the immunochemical method by the liquid sample or another liquid.

7. The method as claimed in claim 1, wherein the salt is added to a solution which contains the analyte and with which the analyte is diluted where appropriate.

8. The method as claimed in at least one of claims 1 to 7, wherein the doubly charged cations are present in a concentration of 30 to 100 mmol/l.

9. The method as claimed in at least one of claims 1 to 7, wherein the doubly charged cations are present in a concentration of 50 to 60 mmol.

## Revendications (Revendications pour l'(les) Etat(s) contractant(s) suivant(s): AT, BE, CH, DE, FR, GB, IT, LI, LU, NL, SE)

1. Utilisation de cations divalents dans un procédé pour la détection immunochimique et pour la détermination immunochimique d'un corps à analyser contenu dans un matériel biologique, pour l'amélioration de la spécificité de la réaction immunochimique, **caractérisée en ce que** des cations divalents, de préférence un sel de calcium et/ou de magnésium, se trouvent à l'état dissous, à une concentration de 5 à 500 mmol/L, lors de la mise en contact du corps à analyser avec un réactif non marqué, moyennant quoi ledit procédé est un ELISA en phase solide.

2. Utilisation selon la revendication 1, **caractérisée en ce que** le sel est le chlorure.

3. Utilisation selon la revendication 1, **caractérisée en ce que** le corps à analyser est un antigène.

4. Utilisation selon la revendication 1, **caractérisée en ce que** le corps à analyser est un anticorps.

5. Utilisation selon la revendication 1, **caractérisée en ce qu'**un corps en réaction est présent sous forme de phase solide.

6. Utilisation selon la revendication 1, **caractérisée en ce que** le sel est contenu sous forme sèche dans une matrice absorbante, et, dans le procédé immunochimique, est mis en solution au moyen de l'échantillon liquide ou d'un autre liquide.

7. Utilisation selon la revendication 1, **caractérisée en ce que** le sel est ajouté à une solution dans laquelle se trouve le corps à analyser, et est éventuellement dilué avec le corps à analyser.

8. Utilisation selon au moins l'une des revendications 1 à 7, **caractérisée en ce que** les cations divalents sont présents dans une concentration de 30 à 100 mmol/L.

9. Utilisation selon au moins l'une des revendications 1 à 7, **caractérisée en ce que** les cations divalents sont présents dans une concentration de 50 à 60 mmol/L.

## Revendications (Revendications pour l'(les) Etat(s) contractant(s) suivant(s): ES)

1. Procédé pour la détection immunochimique et pour la détermination immunochimique d'un corps à analyser contenu dans un matériel biologique, pour l'amélioration de la spécificité de la réaction immunochimique, **caractérisé en ce que** des cations divalents, de préférence un sel de calcium et/ou de magnésium, se trouvent à l'état dissous, à une concentration de 5 à 500 mmol/L, lors de la mise en contact du corps à analyser avec un réactif non marqué, moyennant quoi ledit procédé est un ELISA en phase solide.

2. Procédé selon la revendication 1, **caractérisé en ce que** le sel est le chlorure.

3. Procédé selon la revendication 1, **caractérisé en ce que** le corps à analyser est un antigène.

4. Procédé selon la revendication 1, **caractérisé en ce que** le corps à analyser est un anticorps.

5. Procédé selon la revendication 1, **caractérisé en ce qu'**un corps en réaction est présent sous forme de phase solide.

6. Procédé selon la revendication 1, **caractérisé en ce que** le sel est contenu sous forme sèche dans une matrice absorbante, et, dans le procédé immunochimique, est mis en solution au moyen de l'échantillon liquide ou d'un autre liquide.

7. Procédé selon la revendication 1, **caractérisé en ce que** le sel est ajouté à une solution dans laquelle se trouve le corps à analyser, et est éventuellement dilué avec le corps à analyser.

8. Procédé selon au moins l'une des revendications 1 à 7, **caractérisé en ce que** les cations divalents sont présents dans une concentration de 30 à 100 mmol/L.

9. Procédé selon au moins l'une des revendications 1 à 7, **caractérisé en ce que** les cations divalents sont présents dans une concentration de 50 à 60 mmol/L.
